# EUROPEAN PATENT SPECIFICATION

(11) **EP 3 381 886 B1**
(45) Date of publication and mention of the grant of the patent: **11.12.2019**
(21) Application number: 17193118.1
(22) Date of filing: 26.09.2017
(51) Int. Cl.: C07C 51/12, C07C 57/58, C07D 213/75

(54) **A PROCESS FOR PRODUCING CARBOXYLIC ACIDS FROM TERTIARY ALCOHOLS UNDER CONTINUOUS FLOW**
VERFAHREN ZUR HERSTELLUNG VON CARBONSÄUREN AUS TERTIÄREN ALKOHOLEN UNTER KONTINUIERLICHEM FLUSS
PROCÉDÉ DE PRODUCTION D'ACIDES CARBOXYLIQUES À PARTIR D'ALCOOLS TERTIAIRES À ÉCOULEMENT CONTINU

(30) Priority: 31.03.2017 EP 17164391
(43) Date of publication of application: 03.10.2018
(73) Proprietor: Helsinn Healthcare SA, 6912 Lugano-Pazzallo (CH)
(72) Inventor: FADINI,Luca, 6710 Biasca (CH); FRASCA, Gionata, 6512 Giubiasco (CH); MONGBANZIAMA, Yvan, 1800 Vevey (CH); ALLEMANN, Christophe, 1630 Bulle (CH)
(74) Representative: Zardi, Marco

(56) References cited:
- HERBERT KOCH ET AL: "?ber die Synthese verzweigter Carbons?uren nach der Ameisens?ure-Methode", JUSTUS LIEBIGS ANNALEN DER CHEMIE, vol. 618, no. 1, 26 November 1958 (1958-11-26), pages 251-266, XP55204702, ISSN: 0075-4617, DOI: 10.1002/jlac.19586180127
- HOFFMANN-EMERY FABIENNE ET AL: "Efficient Synthesis of Novel NK1 Receptor Antagonists: Selective 1,4-Addition of Grignard Reagents to 6-Chloronicotinic Acid Derivatives", JOURNAL OF ORGANIC CHEMISTRY,, vol. 71, no. 5, 3 March 2006 (2006-03-03), pages 2000-2008, XP002569709, ISSN: 0022-3263, DOI: 10.1021/JO0523666 [retrieved on 2006-02-09]
- FUKUYAMA, F, MUKAI, Y. RYU, I: "Koch-Haaf reaction of adamantols in an acid-tolerant hastelloy-made microreactor", BEILSTEIN JOURNAL OF ORGANIC CHEMISTRY, vol. 7, 15 September 2011 (2011-09-15), pages 1288-1293, XP002780127,

## Description

### STATE OF THE ART

The carbonylation of alcohols and alkenes has been studied since more than 60 years and it involves the insertion of a carbonyl moiety in an alcoholic substrate. Two main ways have been developed in order to obtain the carboxylic products: the metal catalyst route, (Reppe, W.; Kröper, H.; v. Kutepow, N.; Pistor, H. J., Carbonylierung III. Umsetzung von Alkoholen und offenen Äthern mit Kohlenoxyd zu Carbonsäuren. Justus Liebigs Annalen der Chemie 1953, 582 (1), 72-86) and the acid catalyst way with the first synthesis of Koch and Haaf, which is known as the Koch-Haaf reaction (Koch, H., Carbonsäure as Olefinen, Kohlenoxyd und Wasser. Brenstoff-Chemie 1955, 36(21/22), 321-328).

In the metal catalyst route, the alcohol goes in a catalytic cycle wherein an insertion of carbon monoxide gives the corresponding carboxylic acid after quenching with water. Although efficient, the metal catalyst route presents some features limiting its industrial exploitation: in particular, it requires the use of catalyst metals such as cobalt, rhodium, iridium, ruthenium and palladium which bear a significant environmental impact; the reaction also needs high temperature (>100°C) and pressure (>20bar), i.e. costly process conditions, requiring specially adapted reactors.

The Koch-Haaf reaction is well known when tertiary carboxylic compounds are desired. This reaction uses the degradation of formic acid by a strong acid to generate the carbon monoxide; the latter then reacts with the formed carbocation to give the carboxylic product. The detailed mechanism of this reaction involves five steps, presented in the following scheme:

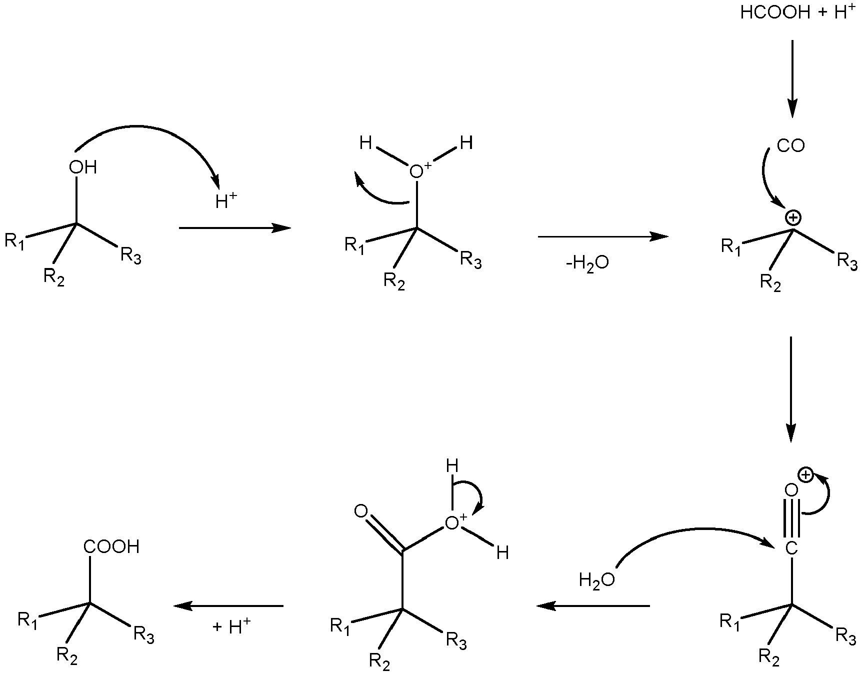

In the first step, the alcohol moiety is protonated by strong acid media and then a water molecule is released, which gives the carbocation formation. Besides catalysing the reaction, the strong acid also allows the in-situ formation of carbon monoxide by the Morgan reaction; the carbon monoxide does a nucleophilic attack on the carbocation, resulting in the formation of a carbonyl moiety (electrophile carbon) on the molecule. Finally, water attacks the carbonyl and, with the loss of a proton, the carboxylic acid is obtained. Problems with the Koch-Haaf reaction have been extensively reported caused by the instability of the carbocation obtained after the second step of the mechanism; as a consequence, besides the desired carboxylic acid, dimerization products and/or other side-products can be synthesized, due to various rearrangements of the carbocation (Koch, H.; Haaf, W., Justus Liebigs Annalen der Chemie 1958, 618 (1), 251-266). For example, by using pentan-2-ol as reactant, three different carboxylic acids have been found : 2-pentanecarboxylic acid, 3-pentancarboxylic acid and 2-methyl-2-butancarboxylic acid (Haaf, W., Chemische Berichte-Recueil 1966, 99 (4), 1149). Strong rearrangement issues have been reported by using various tert-alkyl substituted cycloalkanols (Peters, J. A.; Bekkum, H. V., Recueil 1971, 90, 65-80). Kock and Haaf had already noticed the formation of a carboxylic acids with nine carbons by using 2-butanol as substrate as well as carboxylic acids with eleven carbons when 2-methyl-2-butanol has been used as starting material (Koch, 1958, op.cit). More recently, Hoffmann et al. (Journal of Organic Chemistry 2006, 71 (5), 2000-2008) observed the formation of two dimer products from the synthesis of 2-(3,5-bistrifluoromethylphenyl)-2-methylpropionic acid according to the strong acid used:

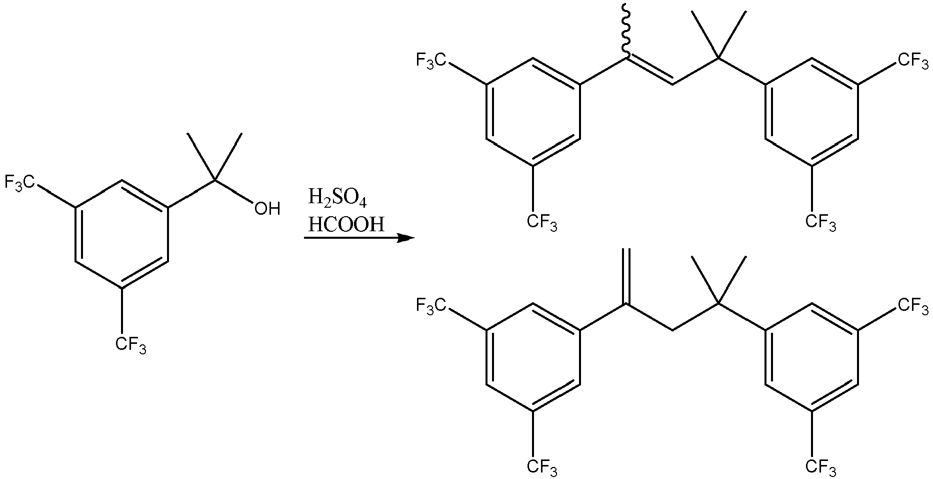

A further issue in the Koch-Haaf reaction consist in the fact that the reactants came from different phases: typically a liquid-liquid-gas system is formed where the alcohol is in the organic phase, the carbon monoxide is in the gas phase, and the strong acid was in the aqueous phase. The obtained multiphase system makes difficult to ensure a uniform contact among the reagents, resulting in difficulties in establishing a reproducible, most effective molar ratios among them. In general, ranges of 2-4 equivalents of HCOOH and 8-12 equivalents of sulfuric acid are used per equivalent of alcohol, the large amount of sulfuric acid being needed to ensure its double role in the formation of the carboxylic acid and then in the formation of carbon monoxide; nevertheless, low yields of the desired product were reported, due to the formation of side-products.

From the years 2000, alternative ways of performing the Koch-Haaf synthesis have been described, replacing the strong acid by acidic ionic liquids as well as solid acid catalyzed such as Nafion-H, silver trifluoromethansulfonate, and H-zeolites : cf: Catal. Commun. 2006, 7 (7), 450-453;. J. Mol. Catal. A: Chem. 2002, 179 (1-2), 271-277; Chem. Commun. (Cambridge, U. K.) 2003, (16), 2070-2071; however the large excess of strong acid, used in large scale, gave a huge amount of acid wastes ; in addition, these process still needed high pressure (50 to 90 bar) as well as high temperature (150 to 200°C). The publication Beilstein Journal of Organic Chemistry, 2011, 7, 1288-1293, describes a Koch-Haaf reaction on adamantanols performed in an acid-tolerant hastelloy-made microreactor.

Overall, the formation of undesired side products remains a significant problem which limits the industrial applicability of Koch-Haaf reaction, as it reduces the yield of the target product and involves high costs for its purification; the Koch-Haaf reaction remains not generally applicable to alcohols in order to obtain the corresponding carboxylic acids at reasonable industrial costs, and suitable solutions remain to be found on a case-by-case basis; at the same time, modifications of the synthesis involving e.g. the use of very high pressures, although interesting on the theoretical viewpoint, are not a convenient alternative for low-cost industrial processes.

### SUMMARY

A new process has now been devised to obtain a carboxylic acid of formula (II) from a tertiary alcohol of formula (I), in which R1, R2 and R3 independently represent an optionally substituted alkyl or optionally substituted aryl group.

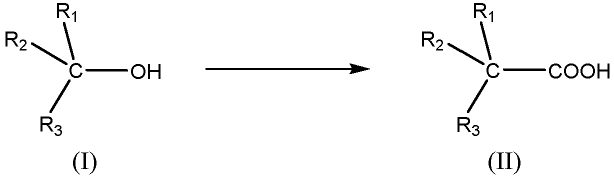

The process is characterized by the reaction of said alcohol (I) with a combination of formic acid with a strong acid, wherein
a) said strong acid is CF₃SO₃H, which is present in an amount of at least 15 molar equivalents per equivalent of compound (I);
b) the reaction is conducted in a microflow reactor at a temperature of at least 50°C, and with a residence time within the reactor of at least 50 seconds.

The maximum amount of equivalents for the strong acid is not critical for the present invention: as a reference, possible maximum values could be, for example 18, 20, 23 or 25 equivalents, per equivalent of compound (I). This synthesis is particularly effective when the tertiary alcohol of formula (I) is a benzylic alcohol derivative, particularly an halogenated one; among them, a preferred compound of formula (I) is 2-[3,5-bis-(trifluoromethyl)phenyl]-propan-2-ol; the corresponding carboxylic acid of formula (II) is a useful intermediate in the synthesis of the NK-1 antagonist compound netupitant.

### DESCRIPTION OF THE FIGURES

Figure 1: Influence of the temperature on the yield of carboxylic derivative (data from Example 1)
Figure 2: Influence of the amount of equivalents of triflic acid on the yield of carboxylic derivative (data from Example 1)

### DETAILED DESCRIPTION

In the above defined formula (I), the substituents R1, R2 and R3 are independently selected from optionally substituted aryl or optionally substituted alkyl groups; preferably said alkyl groups are C1-10 alkyl groups; preferably, said optional substituents are CF₃ groups.

Preferably, the alcohol of formula (I) is a benzylic alcohol, i.e. benzyl alcohol optionally substituted on one or more of its carbon atoms; more preferably the benzyl alcohol contains one or more halogen atoms. According to a more preferred embodiment, the alcohol (I) and the corresponding acid (II) have, respectively, the structures (Ia) and (IIa):

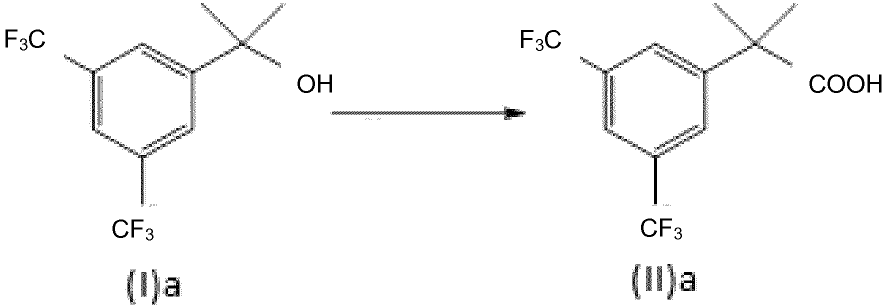

The compound of formula (Ia), 2-[3,5-bis-(trifluoromethyl)phenyl]-propan-2-ol, is also identified herein as 11-NETU; the corresponding carboxylic derivative of formula (II)a is also identified herein as 12-NETU.

The compound of formula (II)a is an intermediate useful for the synthesis of the NK-1 antagonist drug netupitant and derivatives thereof. Accordingly, the process of the present invention is part of a synthetic procedure to obtain netupitant, having the structure (III) and also known as 2-[3,5-bis(trifluoromethyl)phenyl]-N,2-dimethyl-N-[4-(2-methylphenyl)-6-(4-methylpiperazin-1-yl)pyridin-3-yl]propanamide) 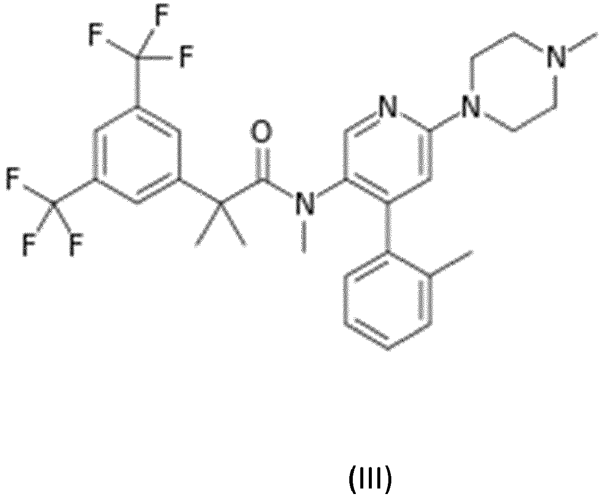 or a derivative thereof. Preferably, said netupitant derivative is a netupitant ester and/or N-oxide derivative. More preferably, the derivative is chosen among the compounds having the following structures:

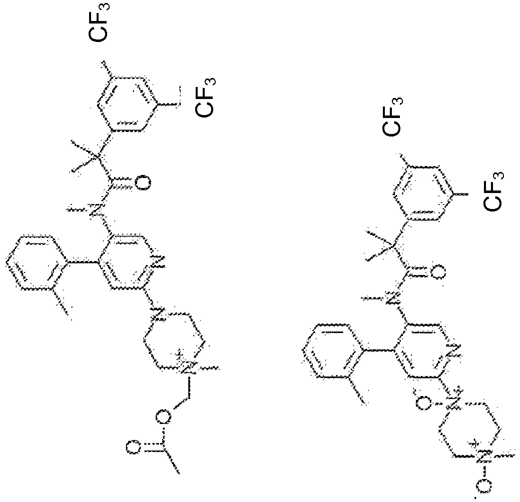

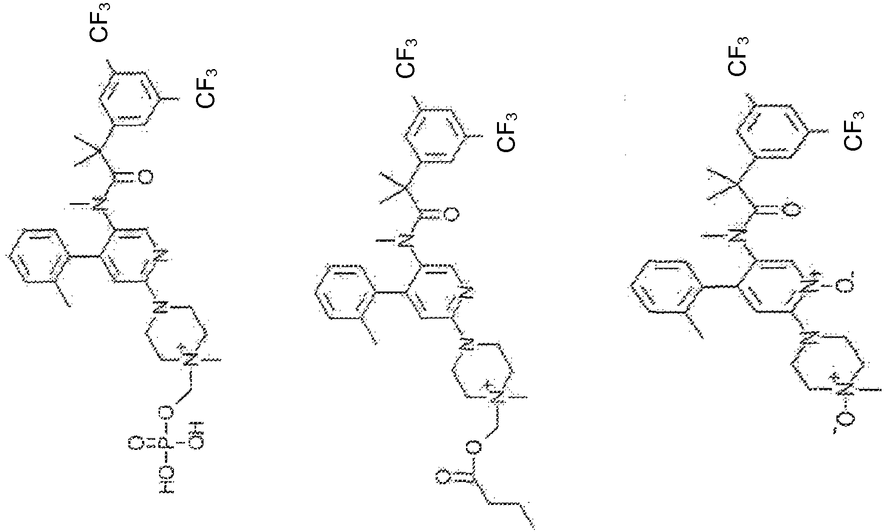

According to the invention, the process is performed in a microflow reactor. Microflow reactors are well-known in the art and are reviewed, for example, in the publication Materials Science and Engineering, Volume 39, No. 2 (2014), pp. 89-101*.* Microflow reactors are also characterizable as continuous flow reactors (as opposed to batch reactors): they include small-section channels (with diameter typically from 100 µm to 1 mm) through which the fluid reagents mixture is pumped. In addition to the standard laminar flow, the small-section of the channels, associated to the channel structure and assembly, promotes turbulent, transversal and/or convection movements in the passing fluid, with the result of a more thorough and quicker homogenization of the reagent mixture, compared to traditional stirring in batch reactors. The above features are especially advantageous when the reagent mixture to be reacted is multiphasic, i.e. particular difficult to mix and homogenize. Typical type of flows generated within these reactors are the "slug flow" (in which a convective mixing movement takes place within sections or "slugs" of the fluid during its flowing through the channels, and the "parallel flow" in which the mixing takes place mainly by diffusion between adjacent flowing phases. Microflow reactors allow an eased, quicker and more reproducibile mass- and heat transfer, and an enhanced control on process parameters; they are normally supplied as modular units which can be easily assembled together to form larger-scale systems, allowing an easy and reproducible scale-up of the process. Examples of microflow reactors are the "Asia-type" reactors (from Syrris Asia), or the reactors H-Cube, O-Cube, X-Cube , X-Cube flash (from ThalesNano Inc.) In order to perform gas-liquid reactions in microflow, several channel designs have been developed, as commonly known in the art. The first design is a simple tube (Teflon, stainless-steel, glass, etc.) where the inner diameter can vary according to the need. In this design, two different flow regimes can be achieved: besides the segmented flow ("slug flow"), an annular flow is generated, wherein a high velocity of gas is used, which pushes the liquid against the wall of the microchannels, resulting in a very thin liquid layer (Angewandte Chemie International Edition 2009, 48 (26), 4744-4746.; Advanced Synthesis & Catalysis 2009, 351 (18), 3260-3268). A further design based on the same principle that the annular flow is the falling film microreactor (Journal of Fluorine Chemistry 2000, 105 (1), 117-128; Chemical Engineering Journal 2015, 262, 1168-1174): the liquid forms a thin layer in micro-channels and flows by gravity, while the gas flows co-currently or counter-currently in front of the liquid phase. A still further design is call mesh microreactor *(*Catalysis Today 2007, 125 (1-2), 34-39; Industrial & Engineering Chemistry Research 2008, 47 (23), 8995-9005; Lab on a Chip 2003, 3 (3), 180-186): this is made of a fine metal or ceramic mesh to separate the liquid flow in several microchannels and gives a continuous contact between the gas and the liquid phase. A further design uses a gas-permeable polymer membrane, which separates the liquid flow and the gas flow. The most popular example is the tube in tube design which use an amorphous fluoropolymer Teflon AF-2400 membrane, which is highly permeable to gases like ozone, hydrogen, oxygen, carbon monoxide, etc. but this membrane has a very low or even no permeability to liquid (Angewandte Chemie International Edition 2011, 50 (5), 1190-1193; Organic Process Research & Development 2015, 19 (7), 812-818): this design can work in the conventional mode with gas outside of the permeable membrane or in reverse mode with the gas inside the membrane (Organic Process Research & Development 2013, 17 (6), 927-933).

Preferred microflow reactors used in the invention are those permitting a segmented flow. A special class of segmented flow reactors, particularly preferred for the purpose of the present invention, has been developed by Corning under the name of Corning® Advanced-Flow™ reactors (AFR): they are described at the following liks: https://www.Corning.com/ worldwide/en/ innovation/Corning-emerging-innovations/advanced-flow-reactors.html;, cf. also http://www.corning.com/media/worldwide/Innovation /documents/ General%20Brochure WEB.pdf (both accessed 30 March 2017). These reactors own a special heart-shaped design of channels. This structure allows a thorough mixing between the phases all along the reactor while keeping a good heat exchange control. In addition of this technology, Corning has developed AFR from lab-scale to the production in order to reproduce the same results from the G1 AFR to the G4 AFR.

According to a further preferred embodiment, said microflow reactor is a coil reactor as described, for example, in: http://www.interchim.fr/cat/ AccessoiresFlowSyn.pdf.; common general knowledge about coil reactors is summarized e.g. by Darvas, Dormán Hessl in "Flow Chemistry", Ed. De Gruyter, 2014, vol. 1, pp.106-108.In the simplest design, the coil reactor is a small-section tube (typically in stainless steel or polymer material like e.g. PTFE, PEEK, PS, ETFE, etc.) with diameters of e.g. 1/32", 1/16" , 1/8", etc.; these systems typically show a high surface-to-volume ratios and are easily modulable in longer sections, or new sections can be added as required for scaling-up. The coil arrangement also allow the setting up of connectable loops through which independent progressive reaction steps take place and/or or separate reagents are added. Coil reactors can also consist in a small section tubing (e.g. 1 mm diameter) would around a metallic "mandrel". In the process according to the present invention, the reaction temperature (intended as bath temperature of the reactor) is higher than 50°C, being preferably comprised between 50 and 80°C. The residence time within the reactor is generally at least 50 seconds, being preferably comprised between 50 and 250 seconds, more preferably between 80 and 200 seconds. Preferably, the process of the invention is performed at a backpressure up to 12 bar, more preferably from 5 to 12 bar.

The Invention is now further described by the following non-limiting examples.

### EXAMPLES

### Example 1 conversion of 2-[3,5-bis-(trifluoromethyl)phenyl]-propan-2-ol (coil reactor variant)

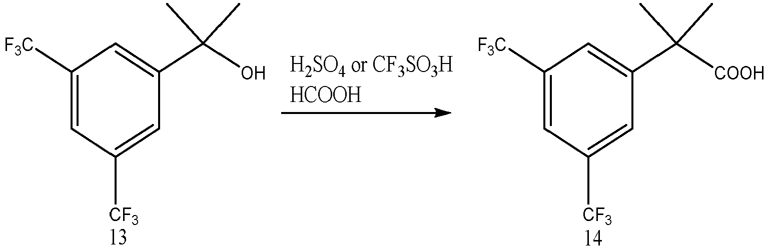

2-[3,5-bis-(trifluoromethyl)phenyl]-propan-2-ol was dissolved in formic acid in an amount suitable to have 6 equivalents of formic acid per equivalent of alcohol (corresponding to a 59 % weight/weight solution). The Koch-Haaf reaction was then performed using either with H₂SO₄ or CF₃SO₃H as strong acid. Whereas only traces of the desired carboxylic derivative (14) have been found with H₂SO₄, the synthesis was successful with CF₃SO₃H and a crude yield of 41% has been found at 50°C for the bath temperature. This last test was performed a second time and a crude yield of 48% was found. The ¹H-NMR spectrum confirmed that the good product has been obtained.
¹H-NMR (300 MHz, Chloroform-d) δ 11.50 (COOH), 7.84 (s, 2H), 7.80 (s, 1H), 1.68 (s, 6H).

Further tests were made to assess the influence on the yield by various parameters like the bath temperature, the pressure in the system, the residence time, the equivalents of strong acid as well as the equivalent of HCOOH and understand which parameters had the most influence on the synthesis. The results of the above tests, carried out between 30 and 55°C and with pressures from 1 to 5 bar are reported in the following table.

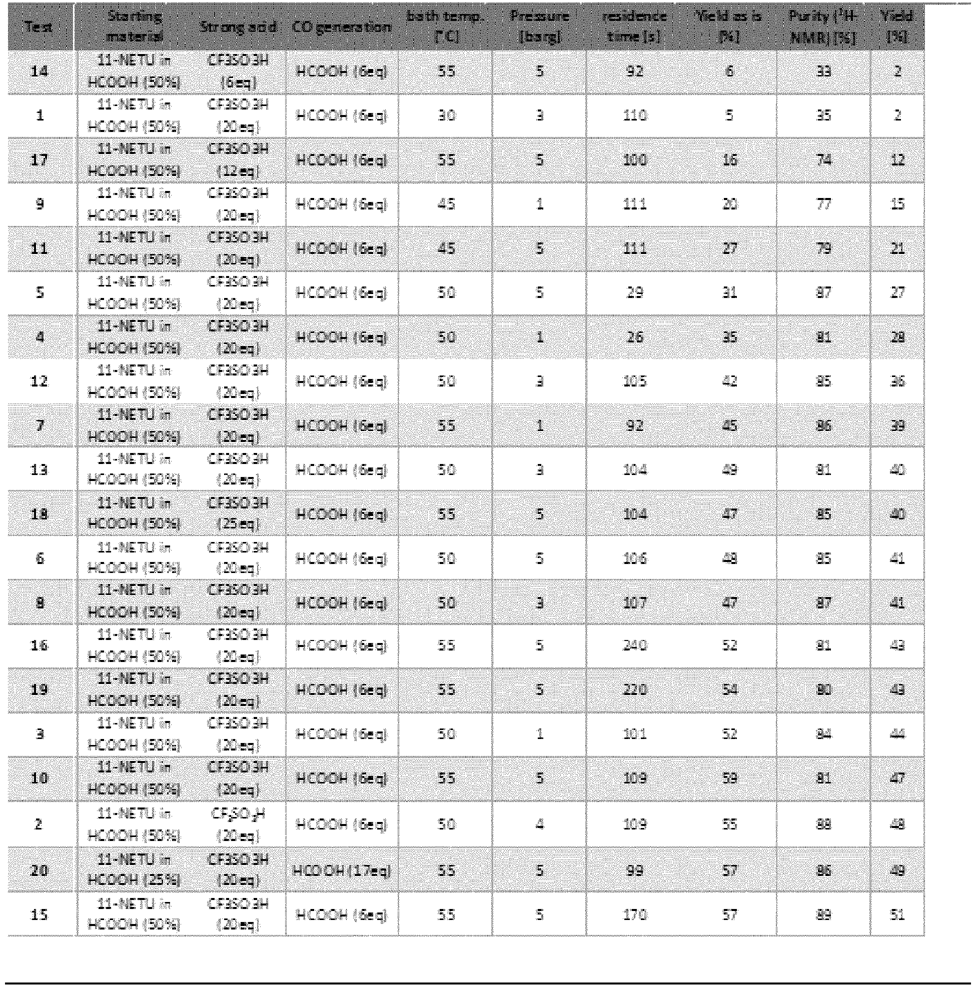

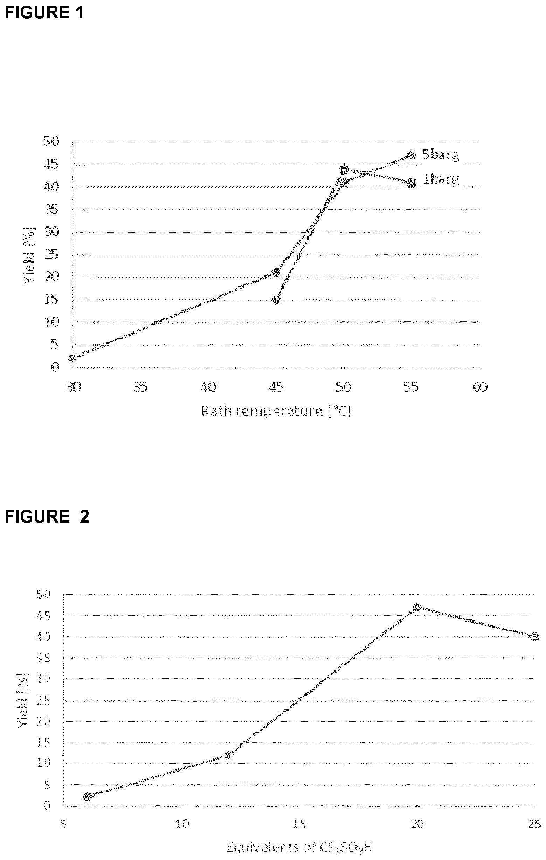

The data in the above table show that the temperature of the bath was one of the most influencing parameters (cf. also Figure 1). In particular, by operating at 50°C or above (and using a sufficient excess of strong acid as discussed below), the reaction yield reached interesting levels in the range of about 40-60%. The pressure had surprisingly no real influence on the yield; a higher pressure value was however preferred as it allowed the system to work at the higher temperatures while keeping a well segmented flow.

Another parameter having a strong influence on the synthesis was the strong acid. Firstly, the choice of the strong acid was very important. The synthesis has been made with the same parameters but by using one time sulfuric acid and one time triflic acid. A yield of 41% was found with CF₃SO₃H, whereas only trace of the carboxylic derivative (14) were obtained with H₂SO₄. Secondly, the equivalents of CF₃SO₃H were also important (cf. also Figure 2). By doing using 6 equivalents (test 14), traces of (14) have been obtained and with two times more equivalents (test 17), the yield increased to only 12%. The amount of 20 equivalents gave normally a yield of 45±5%. These results meant that a large excess of strong acid was needed for this synthesis; at the same time, the use of more than 20 equivalents did not improve the yield any further (test 18). The residence time was found to have influence on the synthesis: with a too short residence time, 30s (tests 4 and 5), the yield was only 25%

In conclusion, the synthesis of netupitant (14) in coil reactor has been successful, giving up 51% of yield. The high solubility of the alcohol (13) in HCOOH allowed a very simple homemade microreactor with 2 pumps, 1 T-mixer, a residence time unit of 8.8ml (Teflon tube) and 1 back-pressure regulator (set-up 6). To obtain interesting yields, the temperature of the bath had to be at least 50°C. For the strong acid, triflic acid was the best results were obtained with 20 equivalents. The pressure had not influence on the yield but a sufficiently high pressure was preferred. The residence time had to be above 50 s, typically between 100s and 170s; a higher time did not increase the yield any further.

### Example 2 Conversion of 2-[3,5-bis-(trifluoromethyl)phenyl]-propan-2-ol (Coring reactor variant)

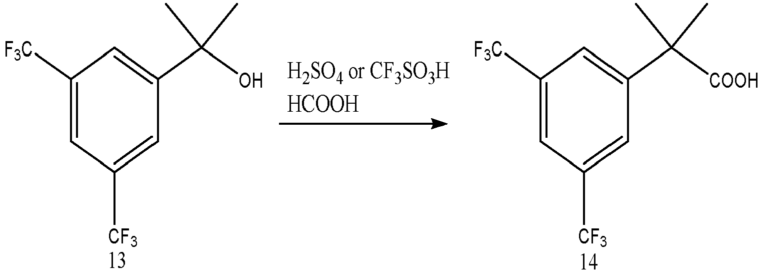

A Low Flow Corning reactor was used in these tests, operated at a pressure of 11 bar. Throughout the conducted tests the temperature was generally kept above 50°C. A residence time higher than 50s was used in all tests. The HCOOH was used in a quantity of 6 equivalents with respect to the alcoholic substrate (13), with the exception of test no. 11 and 12 where the total number of equivalent was respectively 8 and 12). The strong acid used in all tests was CF₃SO₃H, with the exception of tests no. 3 and 8 (using respectively CF₃COOH and H₂SO₄). The test results are summarized in the following Table.

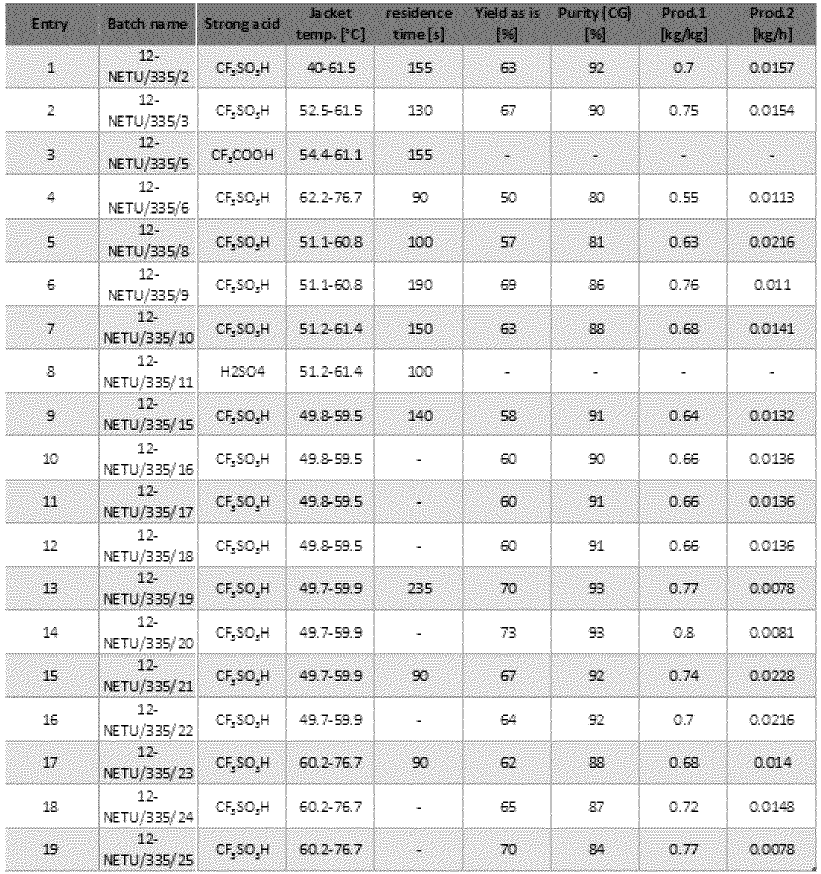

In case of tests no. 3 and 8 (using a strong acid different from CF₃SO₃H, the desired carboxylic derivative (14) could not be obtained due to process problems (insufficient gassing and/or system overpressure). The test all the other cases, the yield was quite high (up to 73%), thus higher than obtained in the coil reactor.

## Claims

1. Process to obtain a carboxylic acid of formula (II) from a tertiary alcohol of formula (I), in which R1, R2 and R3 independently represent an optionally substituted alkyl or optionally substituted aryl group, by reaction of said alcohol (I) with a combination of formic acid with a strong acid, 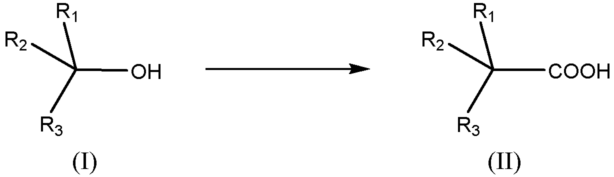 wherein:
a. said strong acid is CF₃SO₃H, present in an amount of at least 15 molar equivalents per equivalent of compound (I);
b. the reaction is conducted in a microflow reactor at a temperature of at least 50°C, and with a residence time within the reactor of at least 50 seconds.

2. Process according to claim 1, wherein said temperature is comprised between 50 and 80°C.

3. Process according to claims 1-2, wherein said residence time is comprised between 50 and 250 seconds, preferably between 80 and 200 seconds.

4. Process according to claims 1-3, performed with backpressure up to 12 bar, preferably from 5 to 12 bar.

5. Process according to claims 1-4, wherein R1, R2 and R3 are independently selected from optionally substituted aryl or C1-10 alkyl groups, said optional substituents being preferably CF₃ groups.

6. Process according to claims 1-5, wherein said alcohol (I) is a benzylic alcohol.

7. Process according to claims 1-6, wherein said alcohol (I) and acid (II) have respectively the structures (Ia) and (IIa):

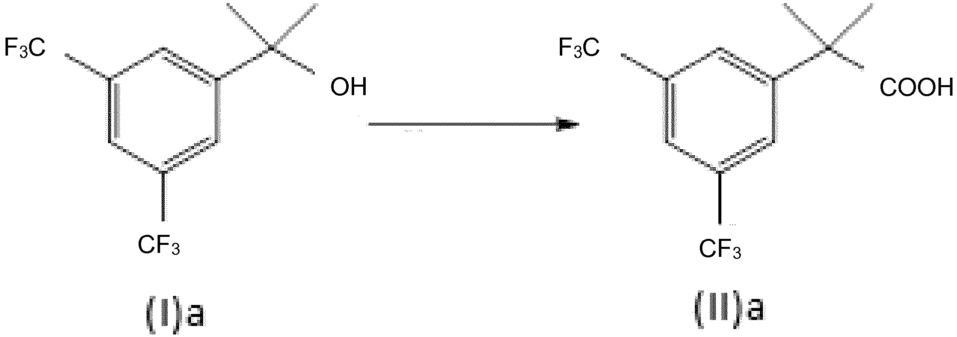

8. Process according to claims 1-7, wherein said reaction is part of a synthetic procedure to obtain a compound of formula (III) (netupitant) or a derivative thereof.

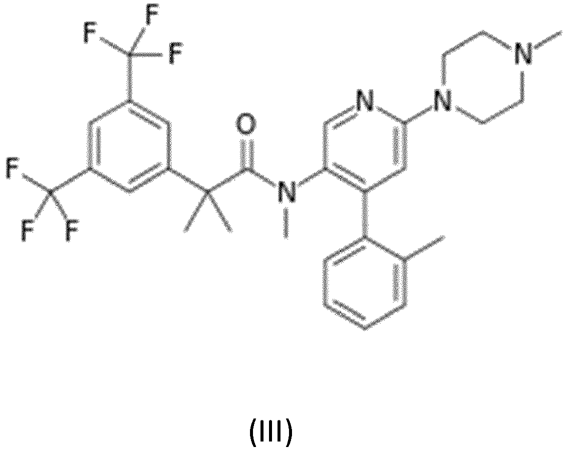

9. Process according to claim 8, wherein said netupitant derivative is chosen among the following:

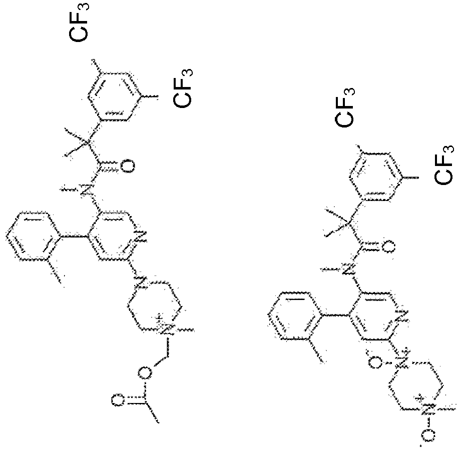

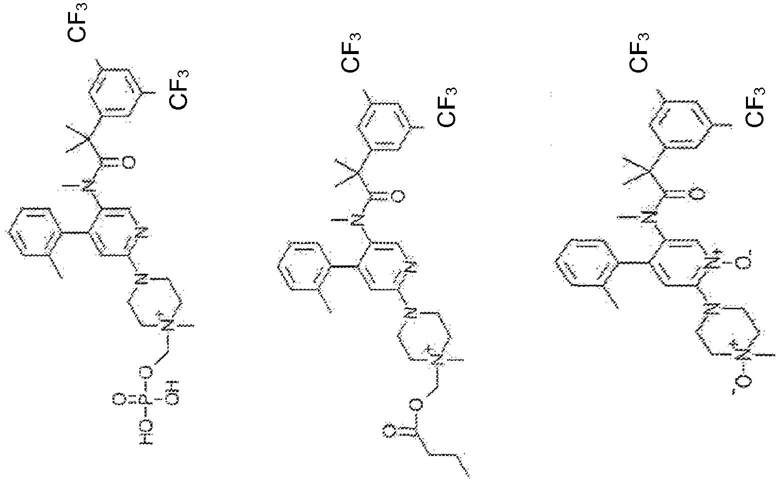

## Patentansprüche

1. Verfahren zum Erhalten einer Carbonsäure der Formel (II) aus einem tertiären Alkohol der Formel (I), worin R1, R2 und R3 unabhängig voneinander eine wahlweise substituierte Alkyl- oder wahlweise substituierte Arylgruppe darstellen, durch Umsetzung des Alkohols (I) mit einer Kombination aus Ameisensäure mit einer starken Säure, 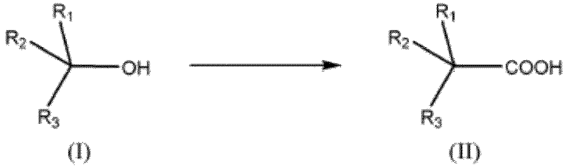 wobei:
a. die starke Säure CF₃SO₃H ist, die in einer Menge von mindestens 15 Moläquivalent pro Äquivalent der Verbindung (I) vorhanden ist;
b. die Reaktion in einem Mikroströmungsreaktor bei einer Temperatur von mindestens 50°C und einer Verweilzeit im Reaktor von mindestens 50 Sekunden durchgeführt wird.

2. Verfahren nach Anspruch 1, wobei die Temperatur zwischen 50 und 80°C umfasst.

3. Verfahren nach den Ansprüchen 1-2, wobei die Verweilzeit zwischen 50 und 250 Sekunden, vorzugsweise zwischen 80 und 200 Sekunden, umfasst.

4. Verfahren nach den Ansprüchen 1-3, durchgeführt mit Gegendruck bis 12 bar, vorzugsweise von 5 bis 12 bar.

5. Verfahren nach den Ansprüchen 1-4, wobei R1, R2 und R3 unabhängig voneinander aus wahlweise substituierten Aryl- oder C1-10-Alkylgruppen ausgewählt sind, wobei die wahlweisen Substituenten vorzugsweise CF₃-Gruppen sind.

6. Verfahren nach den Ansprüchen 1-5, wobei der Alkohol (I) ein Benzylalkohol ist.

7. Verfahren nach den Ansprüchen 1-6, wobei der Alkohol (I) und die Säure (II) jeweils die Strukturen (Ia) und (IIa) aufweisen:

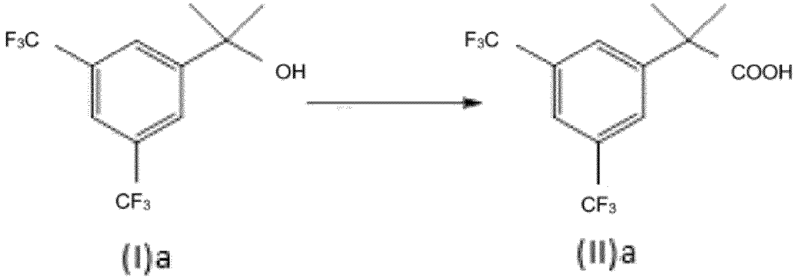

8. Verfahren nach den Ansprüchen 1-7, wobei die Reaktion Teil eines synthetischen Verfahrens ist, um eine Verbindung der Formel (III) (Netupitant) oder ein Derivat davon zu erhalten.

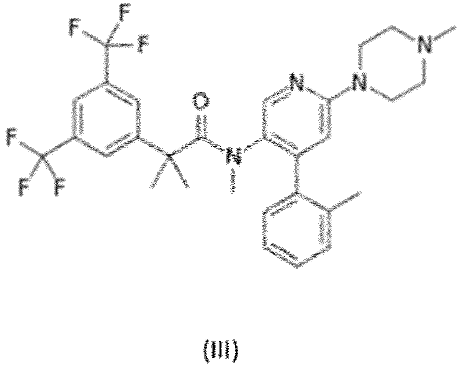

9. Verfahren nach Anspruch 8, wobei das Netupitant-Derivat unter folgenden ausgewählt wird:

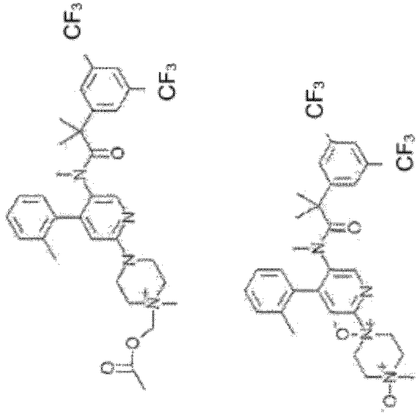

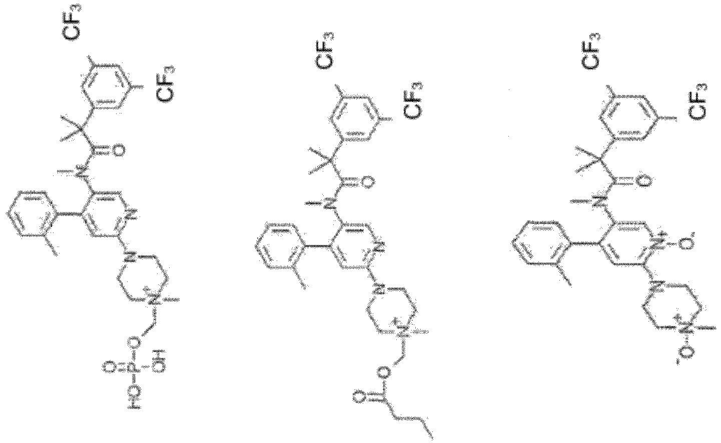

## Revendications

1. Procédé d'obtention d'un acide carboxylique de formule (II) à partir d'un alcool tertiaire de formule (I), dans laquelle R₁, R₂ et R₃ représentent indépendamment un groupe alkyle éventuellement substitué ou aryle éventuellement substitué, en faisant réagir ledit alcool (I) avec une combinaison d'acide formique et d'un acide fort, 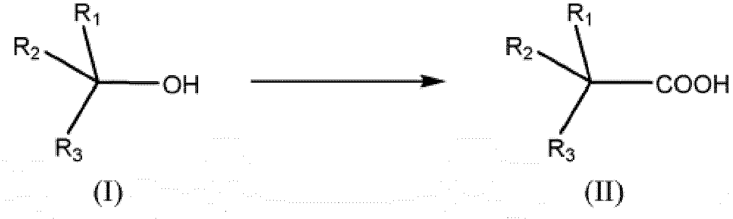 dans lequel :
a. ledit acide fort est CF₃SO₃H, présent en une quantité d'au moins 15 équivalents molaires par équivalent de composé (I) ;
b. la réaction est réalisée dans un réacteur à micro-flux à une température d'au moins 50°C, et avec un temps de résidence à l'intérieur du réacteur d'au moins 50 secondes.

2. Procédé selon la revendication 1, dans lequel ladite température est comprise entre 50 et 80°C.

3. Procédé selon les revendications 1 à 2, dans lequel ledit temps de résidence est compris entre 50 et 250 secondes, de préférence entre 80 et 200 secondes.

4. Procédé selon les revendications 1 à 3, réalisé avec une contre-pression allant jusqu'à 12 bar, de préférence de 5 à 12 bar.

5. Procédé selon les revendications 1 à 4, dans lequel R₁, R₂ et R₃ sont choisis indépendamment parmi les groupes aryle éventuellement substitué ou alkyle en C1-10, lesdits substituants éventuels étant de préférence des groupes CF₃.

6. Procédé selon les revendications 1 à 5, dans lequel ledit alcool (I) est un alcool benzylique.

7. Procédé selon les revendications 1 à 6, dans lequel lesdits alcool (I) et acide (II) ont respectivement les structures (Ia) et (IIa) :

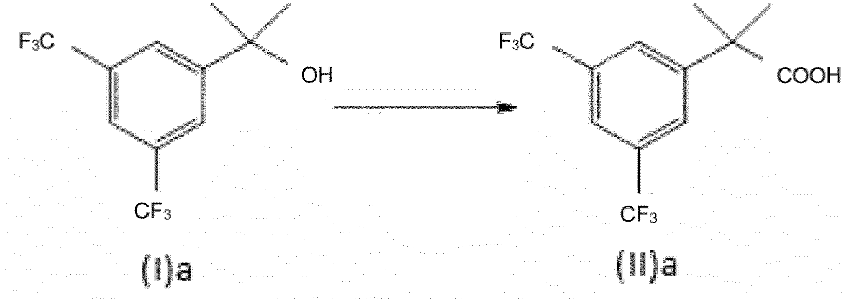

8. Procédé selon les revendications 1 à 7, dans lequel ladite réaction fait partie d'une procédure de synthèse permettant d'obtenir un composé de formule (III) (nétupitant) ou un dérivé de celui-ci.

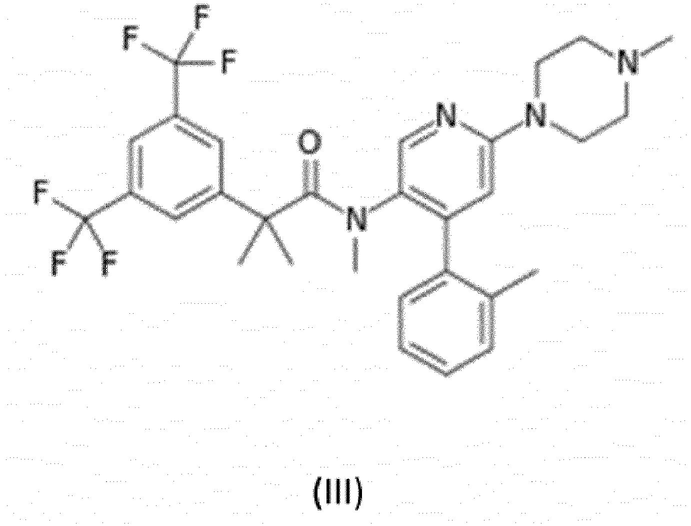

9. Procédé selon la revendication 8, dans lequel ledit dérivé de nétupitant est choisi parmi les suivants :
